# EUROPEAN PATENT SPECIFICATION

(11) **EP 2 200 679 B1**
(45) Date of publication and mention of the grant of the patent: **16.05.2018**
(21) Application number: 08800008.8
(22) Date of filing: 17.09.2008
(51) Int. Cl.: A61M 5/31, A61M 5/315, A61M 5/20

(54) **A RELEASABLE CONNECTING MECHANISM**
LÖSBARER VERBINDUNGSMECHANISMUS
MÉCANISME D'ACCOUPLEMENT AMOVIBLE

(30) Priority: 21.09.2007 AU 2007905184
(43) Date of publication of application: 30.06.2010
(60) Divisional application: 14182680.0
(73) Proprietor: Imaxeon Pty Ltd, Rydalmere, NSW 2116 (AU)
(72) Inventor: NEWING, Timothy, John, Thornleigh NSW 2120 (AU); KHAJEH-TAHERI, Shahab, Cecil Hills NSW 2171 (AU)
(74) Representative: Eisenführ Speiser
(86) International application number: PCT/AU2008/001373
(87) International publication number: WO 2009/036496

(56) References cited:
- EP-A2- 0 308 380
- US-A- 4 677 980
- US-A- 4 677 980
- US-A- 4 908 020
- US-A- 4 911 695
- US-A- 4 911 695
- US-A- 5 007 904
- US-A- 5 007 904
- US-A- 5 383 858

## Description

### FIELD

The invention relates to a releasable connecting mechanism that can be used in a syringe.

### BACKGROUND

In this specification where a document, act or item of knowledge is referred to or discussed, this reference or discussion is not an admission that the document, act or item of knowledge or any combination thereof was at the priority date, publicly available, known to the public, part of common general knowledge; or known to be relevant to an attempt to solve any problem with which this specification is concerned.

In contrast injectors, replaceable syringes are used to deliver a liquid contrast media into the body of a patient. The contrast media travels in the patient's blood vessels to enable the location of the blood vessels, and any blockage in the vessels, to be detected using an X-ray, computed tomography (CT), or magnetic resonance imaging (MRI) scanner.

A syringe in this application consists of a moving element (the plunger), which propels the contrast media, and a container (the syringe barrel), which forms the body of the syringe assembly. Contrast media is typically in liquid form. The contrast media in a syringe may leak past the seal formed between the plunger and the syringe barrel. If the contrast media is left to dry, it forms a hard solid residue. This can be a problem for automated injector systems, particularly those that use replaceable syringes. In such systems, the plunger in the syringe releasably connects to a piston driven by the system, which pushes the plunger along the syringe to inject the contrast media into a patient. Any leak of contrast media past the plunger (such as during use or due to malfunction of the seal of the plunger) may come into contact with the part connecting the plunger to the piston. If the contrast media is allowed to dry, the plunger may become stuck to the piston, which makes maintenance difficult and expensive. If contrast media leaks into the connecting part of the plunger before it connects to the piston, the solid residue makes it difficult for the piston and plunger to engage or function properly, especially if the connecting part involves a mechanism with moving parts, such as retractable locking pins.

It is therefore desired to address one or more of the above, or to at least provide a useful alternative, preferably with a minimum of moving parts.

The EP 0 308 380 A2 discloses a self-destroying hypodermic syringe in which a cylindrical member is provided with two equal sets of grooves arranged in a zigzag pattern which comprises first groove setting at a free edge and extending obliquely toward plunger. If efforts are made to pull the plunger upwards after use of the syringe, pins will pass out at open ends of a second groove. Due to a freely turnable member, it will not be possible to manipulate the syringe to make the pins re-enter the first groove by pulling out the plunger rod.

Furthermore, the US 4 677 980 A discloses an angiographic syringe with a plunger that is releasably connected to a driving mechanism for an injecting operation, and released from the driving mechanism when the injection operation has been completed, such that the driving mechanism can be withdrawn from the syringe relative to the plunger without drawing body fluid of a patient into the syringe.

Additionally, the US 4 911 695 A and the US 5 007 905 disclose a plunger having a generally converging distal portion, and an approximate face on which is mounted a coupling structure. The coupling structure is transversely engagable by, and transversally disengageable from, a driving mechanism of a power-driven angiographic syringe, and once engaged, cannot be disengaged by rotation of the driving mechanism relative to the plunger in the absence of a transfer translational movement of the driving mechanism and plunger relative to one another.

### SUMMARY

According to the present invention, there is provided a releasable connecting mechanism for a syringe, said mechanism including:
a piston having a head portion with one or more radially projecting pins, said head portion being rotatable with said pins about an axis along said piston;
a plunger body having a recess formed within said body for receiving said head portion, said recess including one or more inwardly projecting flanges for guiding said pins to rotate said head portion relative to said body;
wherein, when said head portion is inserted into said recess, said flanges rotate said head portion to a locking position where said body resists disengagement from said piston, and when said head portion is further inserted into said recess, said head portion is released from said locking position and rotates to a release position allowing said piston to disengage from said body.

The present invention also provides a releasable connecting mechanism for a syringe, said mechanism including:
a piston having a head portion with one or more outwardly projecting flanges, said head portion being rotatable with said flanges about an axis along said piston;
a plunger body having a recess formed within said body for receiving said head portion, said recess including one or more inwardly projecting pins for guiding the rotation of said head portion relative to said body;
wherein, when said head portion is inserted into said recess, said pins rotate said head portion to a locking position where said body resists disengagement from said piston, and when said head portion is further inserted into said recess, said head portion is released from said locking position and rotates to a release position allowing said piston to disengage from said body.

### BRIEF DESCRIPTION OF THE DRAWINGS

Representative embodiments of the present invention are herein described, by way of example only, with reference to the accompanying drawings, wherein:
Figures 1 to 3 are block diagrams of one embodiment of the connecting mechanism;
Figures 4 to 6 are cross-sectional diagrams of one embodiment of the connecting mechanism at different stages of operation;
Figures 7 to 10 are diagrams of a top concave member of a plunger;
Figures 11 to 18 are diagrams of a bottom ring member of a plunger;
Figures 19 to 22 are diagrams of a rotatable head portion of a piston;
Figures 23 to 34 are diagrams of an example useful for understanding the invention of the connecting mechanism at different stages of operation;
Figures 35 to 39 are diagrams of an example useful for understanding the invention of a rotatable head portion;
Figures 40 and 41 are bottom views of a rotatable head portion;
Figures 42 to 49 are different views of a representative embodiment of the head portion; and
Figures 50 to 58 are views of a representative embodiment of the plunger as assembled.

### DETAILED DESCRIPTION OF THE REPRESENTATIVE EMBODIMENTS

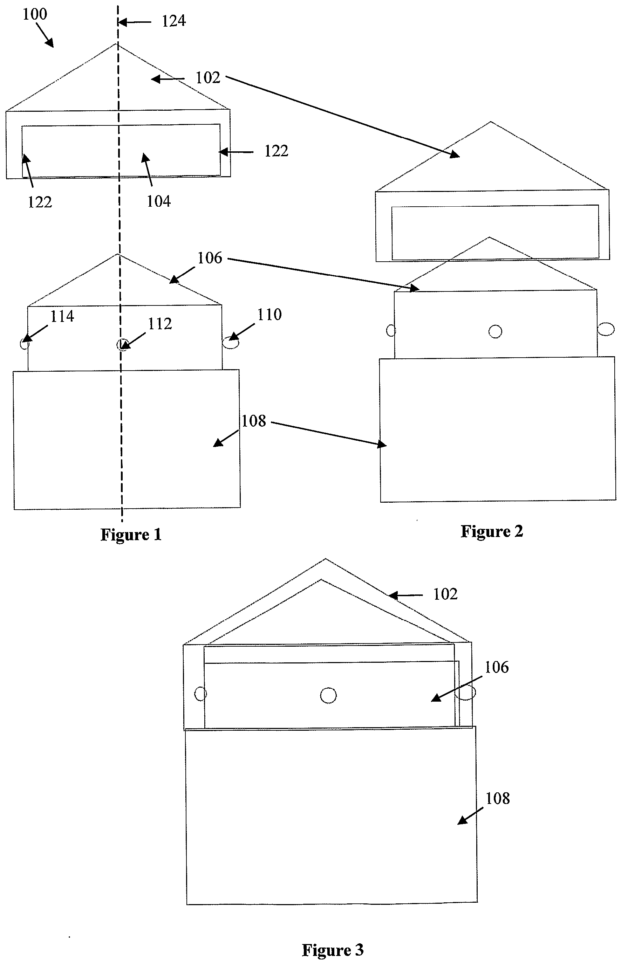

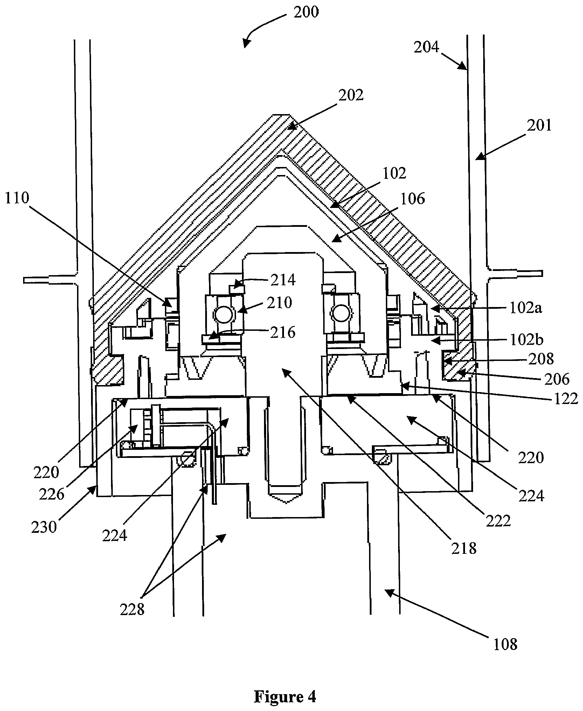

Figure 1 shows the components 100 operating inside the main chamber 200 of a syringe barrel 201 (see Figure 4), which includes a plunger body 102 and a piston 108 with a rotatable head portion 106. A resilient cap 202 (see Figure 4), which is preferably made of an elastic material such as rubber, is fitted over the plunger body 102 to form a seal with the inner surface 204 of the main chamber 200 of the syringe barrel 201. The main chamber 200 of the syringe barrel 201 stores a liquid, such as contrast media. During use, the head portion 106 of the piston 108 connects with the plunger body 102, so that the plunger body 102 can be pushed along the main chamber 200 by the piston 108 to expel any substance (e.g. contrast media) stored in the chamber 200. The connecting mechanism refers to the mechanical interface between two components (e.g. the plunger body 102 and the head portion 106 of the piston 108), which enables these components to be releasably connected to each other.

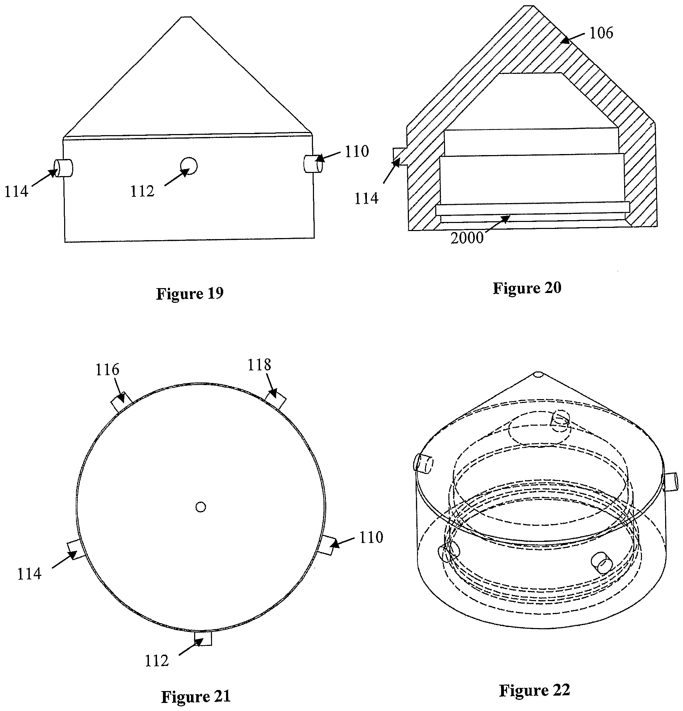

As shown in Figure 1, the piston 108 has a head portion 106 with one or more radially projecting pins 110, 112, 114, 116 and 118 (which, preferably, are radially and evenly spaced from each other, as shown in Figure 21). The head portion 106 is rotatable together with the pins 110, 112, 114, 116 and 118 about an axis 124 along the length of piston 108.

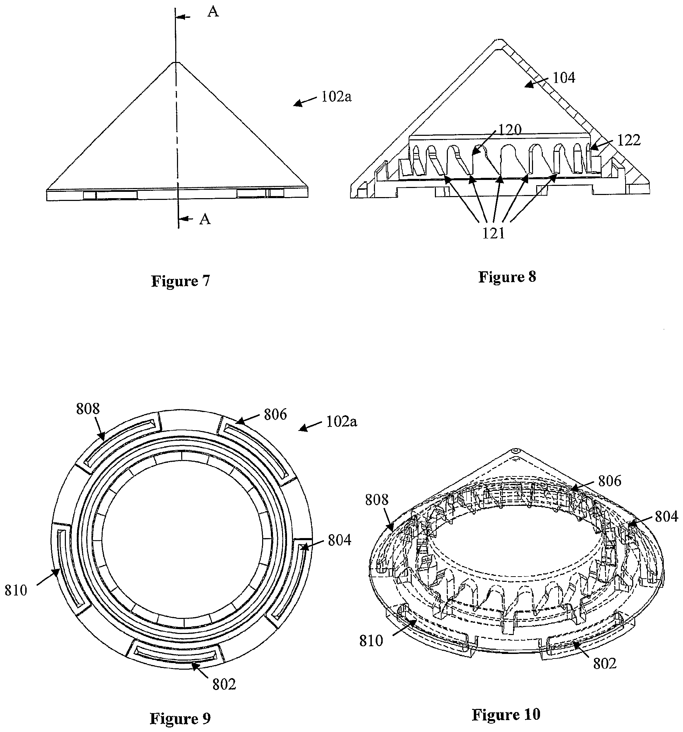

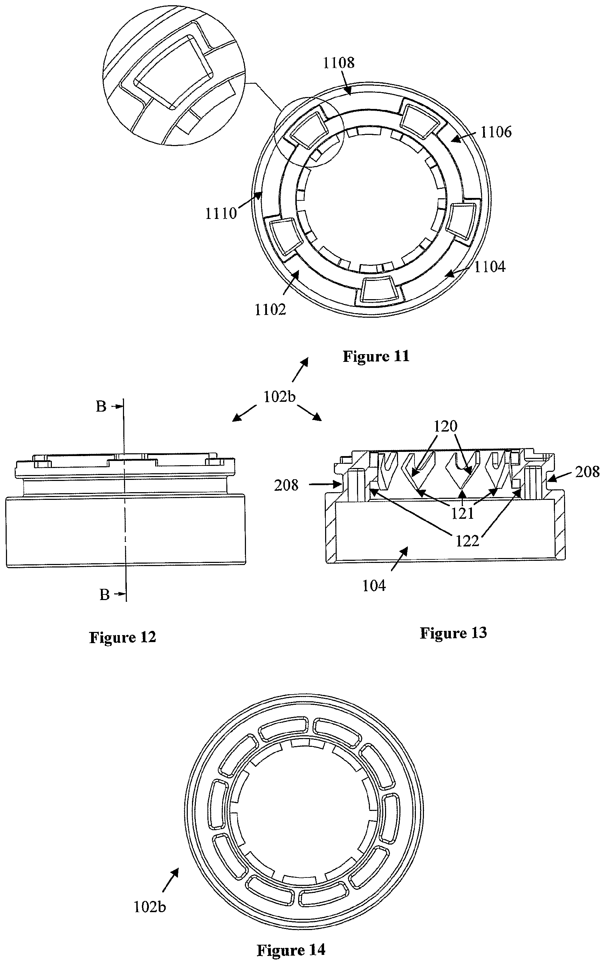

As shown in Figures 8 and 13, the plunger body 102 has a recess 104 formed within the body 102 for receiving the head portion 106. The recess 104 includes one or more inwardly projecting flanges 120 on the inner wall 122, where the edges of the flanges 120 define a path for guiding the movement of the pins 110, 112, 114, 116 and 118 to rotate the head portion 106 relative to (and inside of) the body 102.

In a representative embodiment, the connecting mechanism includes an odd number of pins 110, 112, 114, 116 and 118. In a representative embodiment, the connecting mechanism has 3 pins in total. In another representative embodiment, the connecting mechanism has 5 pins in total. The pins are preferably located on the head portion 106, or in another representative embodiment, are included as part of the plunger body 102.

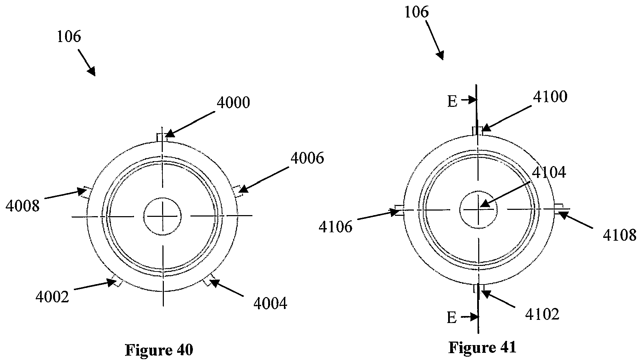

Some of the advantages from having an odd number of pins 110, 112, 114, 116 and 118 in the connecting mechanism (e.g. on the head portion) can be better understood from the examples shown in Figures 40 and 41. Figures 40 and 41 respectively show the top views of different head portions 106 with 5 and 4 radially spaced pins 4000, 4002, 4004, 4006, 4008, and 4100, 4102, 4106, 4108. If contrast media has been deposited onto one or more of the pins 4000, 4002, 4004, 4006 and 4008, and is allowed to dry or harden, the effective diameter of the contrast encrusted pins 4000, 4002, 4004, 4006 and 4008 would increase and become more likely to interfere with the cross section of the path (defined by the flanges 120) during engagement of the plunger body 102 and the head portion 106.

As shown in Figure 41, a head portion 106 with a 4-pin configuration has two natural axes of rotation, since each of the pins 4100, 4102, 4106 and 4108 has a corresponding opposing pin, which can be referred to as a pin pair. Such natural axes will exist in connecting mechanisms that have an even number of evenly spaced pins. In Figure 41, the head portion 106 normally moves in an upward direction (i.e. moving out of the page towards the reader) and rotates anti-clockwise to engage with the plunger body 102 (not shown in Figure 41). However, if a single pin of a pin pair becomes contrast encrusted (e.g. 4100), the contrast encrusted pin would initially cause some resistance to the engagement of the encrusted pin (and the head portion 106) with the plunger body 102. This may cause rotational moment to occur about the axis E-E of the perpendicular pin pair 4100 and 4102 through a central pivot point 4104 of the whole rotating piston interface bearing (e.g. pin 4100 is pushed in a direction heading into the page, which results in pin 4102 being pushed in an opposite direction heading out of the page). The other pins 4102, 4106 and 4108 that are not contrast encrusted are properly received into the respective paths (defined by the flanges 120) of the plunger body 102. These pins 4102, 4106 and 4108 exert torque that helps rotate the head portion 106 relative to the plunger body 102. When the head portion 106 is rotated in this way, the contrast encrusted pin 4100 is forced to rotate with the other pins 4102, 4106 and 4108, which forces the encrusted pin 4100 into its corresponding path in the plunger body 102. The edges of the flanges 120 that define the path help push away the contrast around the encrusted pin 4100. It can be appreciated that by having a greater number of pins 4110, 4102, 4106 and 4108, there are a greater number of places from which torque can be exerted to rotate the head portion 106.

Figure 40 relates to a head portion 106 with a 5-pin configuration that operates in the same way as the head portion 106 shown in Figure 41. The arrangement of pins 4000, 4002, 4004, 4006 and 4008 have no natural axis of rotation of the type as described with reference to in Figure 41. The result is that should a single pin 4000 become encrusted with contrast media, the opposite pin "pair" 4002 or 4004 will tend to distribute and dissipate the rotational moment of that single pin 4000. Each pin of the 5-pin version of the head portion 106 has at least one opposite pin "pair" in a triangle formation. In the example shown in Figure 40, each pin 4000, 4002, 4004, 4006 and 4008 has two other opposing pins that guide the rotation of the pin, which assists the pin to be received into the path (defined by the flanges 120) in the plunger body 102 in circumstances such as where the pin is contrast encrusted.

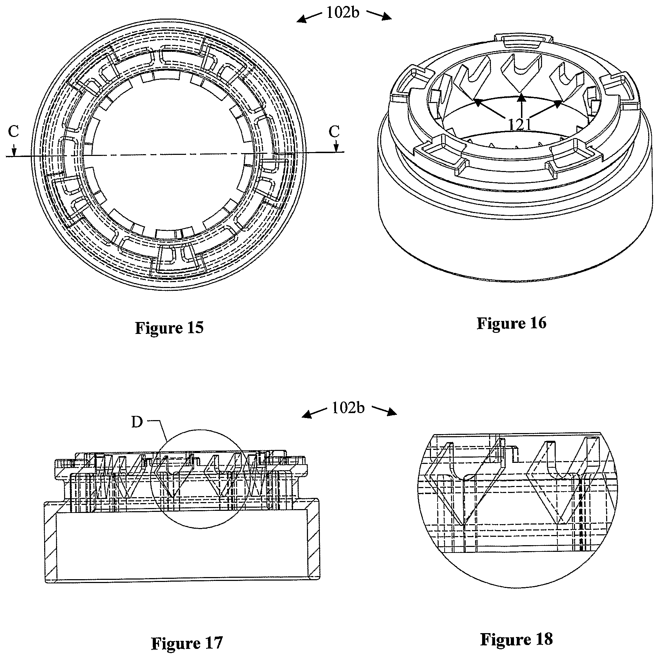

The interface of the connecting mechanism between the head portion 106 and the plunger 102 typically requires that all pins 110, 112, 114, 116 and 118 enter the path defined by the flanges 120 together (i.e. at the same time) to commence the engagement of the plunger body 102 to the head portion 106. As the head portion 106 is received into the plunger 102, all of the pins 110, 112, 114, 116 and 118 may come into contact with the lead-in path apex points 121 (see Figures 13 and 16) simultaneously, thus resulting in a pushing action on the plunger 102 rather than a rotation of the head portion 106. This problem may occur regardless of the number of pins 110, 112, 114, 116 and 118 on the head portion. Typically, however, the apex 121 of the flanges 120 on the plunger 102 are quite small, and the diameter of the pins 110, 112, 114, 116 and 118 are considerably larger than the apex 121 dimensions. Accordingly, the likelihood of this problem occurring can be reduced by ensuring that there are very tight tolerances (e.g. of the order of 0.05 mm) in the positioning of the apex points 121 to minimise the exact matching of the pin 110, 112, 114, 116 and 118 centrelines and lead-in path apex points 121.

In a representative embodiment, the entire head portion 106 is received by the recess 104, so that the mechanical interlocking arrangement between the flanges 120 and the pins 110, 112, 114, 116 and 118 operate wholly inside of the plunger body 102. This enclosed interlocking arrangement reduces the exposure of the flanges 120 and pins 110, 112, 114, 116 and 118 to substances stored inside of the syringe barrel 201 (e.g. contrast media) that may leak past the resilient cap 202. For instance, the enclosed configuration minimises the ability for contrast media to come into contact with the flanges 120 and pins 110, 112, 114, 116 and 118, which in turn, reduces the quantity of dried (or solid) contrast media that may jam or interfere with the mechanical interlocking arrangement between the flanges 120 and pins 110, 112, 114, 116 and 118 of the head portion 106 and plunger body 102. The enclosed interlocking arrangement is further enhanced by the piston's support member 224, which has a load bearing surface 222 that sits flush against a shoulder 220 of the plunger body 102 to substantially prevent the flow of a substance in the syringe (e.g. contrast media) into the space where the pins 110, 112, 114, 116 and 118 connects with the flanges 120.

After the piston 108 is released from the plunger body 102, the plunger body 102 preferably remains in the chamber 200 of the syringe barrel 201 so that the plunger body 102 can be disposed together with the syringe barrel 201. The piston 108 may be part of an injector machine that is not replaced after use with a syringe 201. During use, the substance in the chamber 200 of the syringe barrel 201 may leak past the cap 202. For example, any contrast media that leaks past the cap 202 as the syringe is being removed may come into contact with the pins 110, 112, 114, 116 and 118 (or flanges 120) on the rotating head portion 106 of the piston 108 and leave behind a solid residue on these parts (if allowed to dry). This residue makes is difficult for the head portion 106 to engage or connect to the plunger body 102 of another syringe barrel 201. The small size of the pins 110, 112, 114, 116 and 118 (or flanges 120) on the head portion 106 also make it difficult to clean these parts (e.g. by hand).

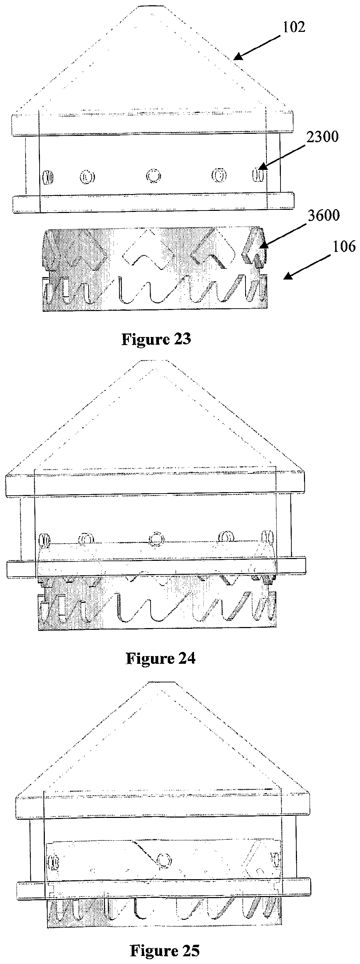

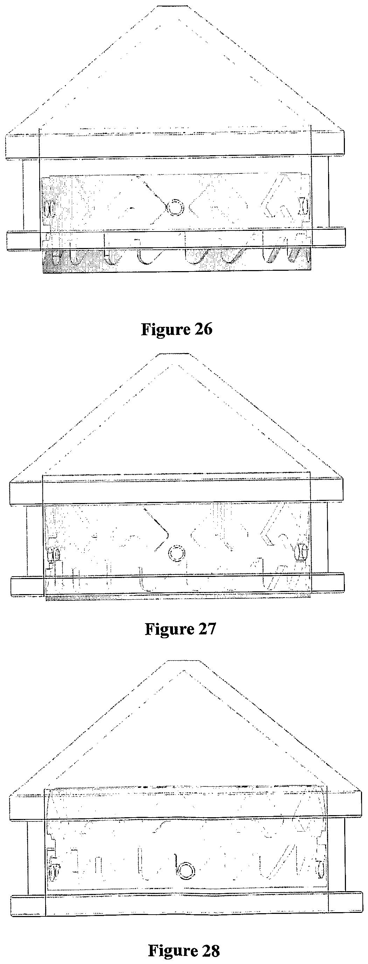

The plunger body 102 is made from a rigid material (such as polycarbonate or similar blended materials), and the recess 104 is preferably shaped to provide a close fit with the outer facing surface of the head portion 106. In the embodiment shown in Figures 1-3
and in the example useful for understanding the invention shown in Figures 23-34, the head portion 106 is smaller in diameter than the plunger body 102, so that the head portion 106 fits into the recess 104 of the plunger body 102 from behind. This configuration allows the plunger body 102 to push away from the head portion 106 any dried contrast media (or any other residue) on the head portion 106 when it is being received into the recess 104. In a representative embodiment, the plunger body 102 is shaped to enable the removal (or cleaning) of solid residue attached onto the head portion 106 as the two parts move towards each other for coupling together.

Figures 2 to 3 show the same component in Figure 1 in different releasable coupling configurations. In Figure 2, the plunger body 102 and piston 108 is shown moving towards each other for engagement. In Figure 3, the plunger body 102 engages the head portion 106 and connects to the piston 108.

Figures 7 to 18 are drawings of various views of the components of the plunger body 102 according to a representative embodiment. The plunger body 102 is made up of a top concave member 102a (as shown in Figures 7 to 10) that fits on top of a bottom ring member 102b (as shown in Figures 11 to 18).

Figure 7 is a side view of the top member 102a. Figure 8 is a cross-sectional view of the top member 102a along section A-A in Figure 7. Figure 9 is a bottom view of the top member 102a shown in Figure 7. Figure 10 is a perspective view of the top member 102a shown in Figure 7, where the dotted lines represent the edges inside of the top member 102a that are hidden from direct view. As shown in Figure 8, the top member 102a is shaped to define a portion of the recess 104, and also defines a portion of the inner wall 122 and flanges 120.

Figure 11 is a top view of the bottom member 102b shown in Figure 12. Figure 12 is a side view of the bottom member 102b. Figure 13 is a cross-sectional view of the bottom member 102b along section B-B in Figure 12. Figure 14 is a bottom view of the bottom member 102b shown in Figure 12. Figure 15 is another top view of the bottom member 102b shown in Figure 12, where the dotted lines represent edges of the bottom member 102b that are hidden from direct view. Figure 16 is a perspective view of the bottom member 102b shown in Figure 12. Figure 17 is another cross-sectional view of the bottom 30 member 102b along section C-C in Figure 15, where the dotted lines represent the edges of the bottom member 102b that are not in direct view. Figure 18 is a more detailed view of Detail D in Figure 17. As shown in Figure 13, the bottom member 102b is shaped to
define a portion of the recess 104, and also defines a portion of the inner wall 122 and flanges 120.

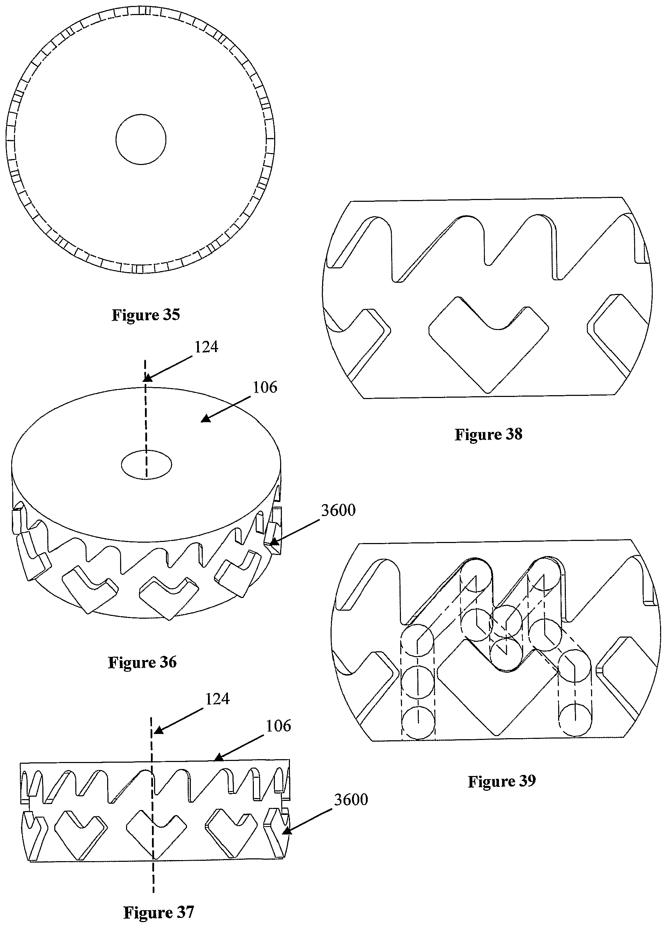

As shown in Figures 9 and 10, the lower portion of the top member 102a has one or more locating members 802, 804, 806, 808 and 810 that are radially and evenly spaced from each other along a lower perimeter of the top member 102a. The locating members 802, 804, 806, 808 and 810 are received in reciprocally shaped locating recesses 1102, 1104, 1106, 1108 and 1110, so that the top member 102a is able to form an interlocking engagement with the bottom member 102b to minimise rotation of the top and bottom members 102a and 102b relative to each other. When the top and bottom members 102a and 102b are coupled to each other, the flanges 120 on both of these members 102a and 102b combine to define a path shaped (see Figure 38) for guiding the movement of the pins 110, 112, 114, 116 and 118 (relative to the plunger body 102) along a saw tooth path in one direction (e.g. as shown in Figure 39). In another representative embodiment, the locating members 802, 804, 806, 808 and 810 are formed on the bottom member 102b, and the recesses 1102, 1104, 1106, 1108 and 1110 are formed on the top member 102a.

Figures 19 to 22 are diagrams of a rotatable head portion 106 of the piston 108. The head portion 106 is shaped to enable it to be received (and preferably wholly received) inside of the recess 104 of the plunger body 102. As shown in Figure 20, the head portion 106 has a cavity 2000 for engaging an end portion of the piston 108.

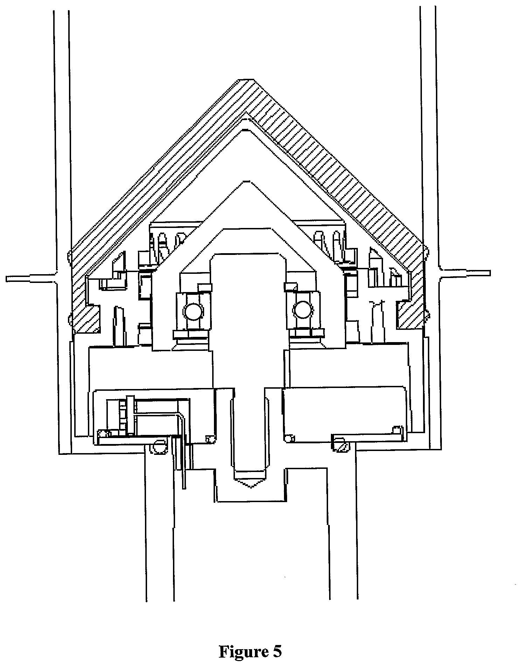

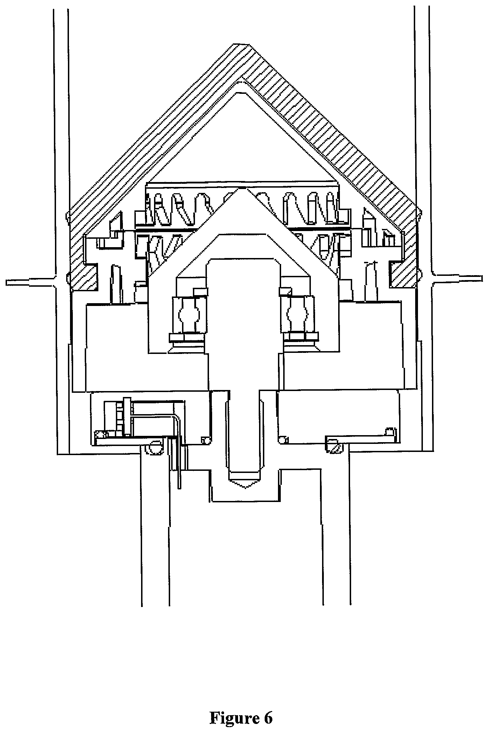

Figures 4 to 6 are cross-sectional diagrams showing a representative embodiment of the connecting mechanism at different stages of operation. Figure 4 shows the head portion 106 received wholly inside of the plunger body 102, and where the plunger body 102 and head portion 106 are in the fully engaged position (e.g. during injection). Figure 5 shows the position of the pins 110, 112, 114, 116 and 118 engaging the flanges 120 for filling. Figure 6 shows the head portion 106 fully disengaged from the plunger body 102.

As shown in Figure 4, a resilient cap 202 is fitted over the outer portion of the top and bottom members 102a and 102b. The rim 206 of the cap 202 is shaped to fit securely into a groove 208 (or recess) formed around the outer circumference of the plunger body 102 (and preferably, on the bottom ring member 102b). The top and bottom members 102a and 102b are held together by the cap 202 that clamps onto the groove 208. On a representative embodiment, the top and bottom members 102a and 102b are also glued or welded together.

As shown in Figure 4, the head portion 106 rotates about a support pin 218. Preferably, the support pin 218 is threaded at one end, and screws into (and extends from) an end portion of the piston 108. Inside the cavity 2000 of the head portion 106, the head portion 106 is coupled to a bearing 210, and the bearing 210 is rotatably coupled to the support pin 218. This enables the head portion 106 to freely rotate about the support pin 218. The bearing 210 is held in place by clip members 214 and 216 (e.g. circ-clips) located above and below the bearing 210. The bearing 210 forms a seal that minimises liquid (e.g. contrast media) from entering into the space between the head portion 106 and the support pin 218. This reduces the risk that contrast media may be allowed to dry in the space between the head portion 106 and the support pin 218, which would give rise to mechanical jamming between these parts 106 and 218.

When the head portion 106 is inserted into the recess 104, the flanges 120 rotate the head portion 106 to a locking position where the plunger body 102 resists disengagement from the piston 108. When the head portion 106 is further inserted into the recess 104 (from the locking position), the head portion 106 is released from the locking position and is allowed to rotate to a release position that allows the piston 108 to disengage from the plunger body 102.

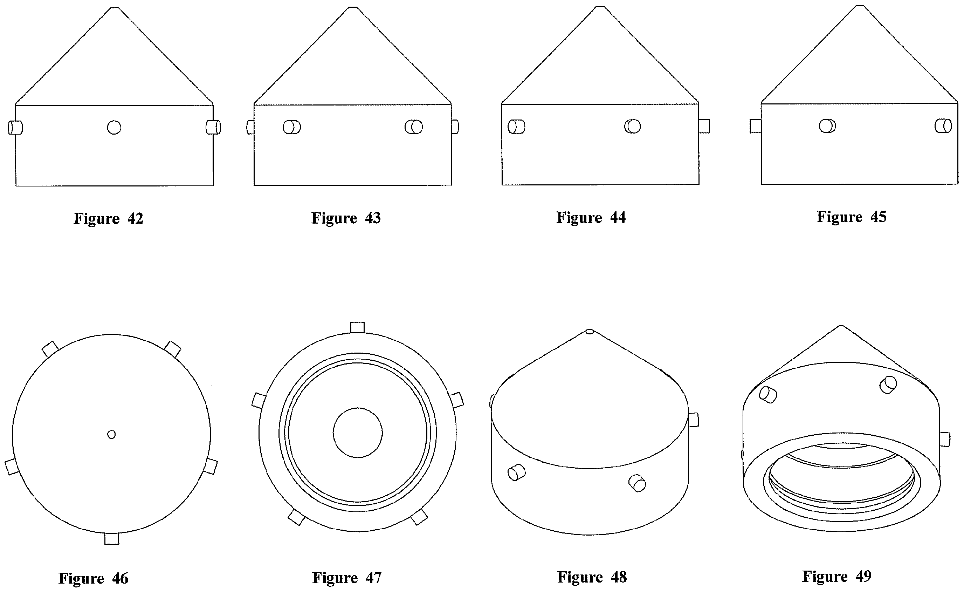

Figures 42 to 49 are different views of a representative embodiment of the rotating head portion 106 of the piston 108. In particular, Figures 42 to 49 respectively show the front view, back view, left side view, right side view, top view, bottom view, upper isometric view and lower isometric view of a representative embodiment of the head portion 106.

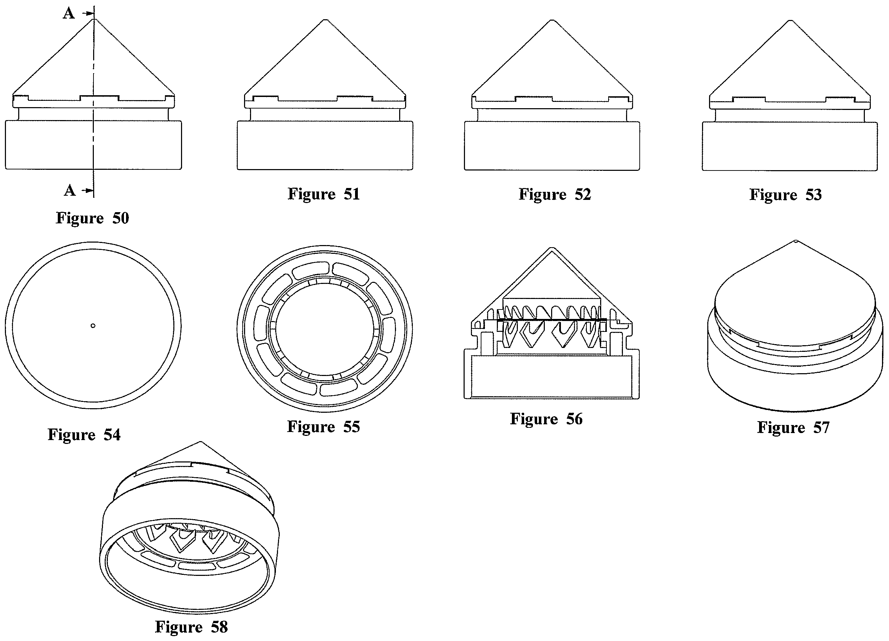

Figures 50 to 58 are views of a representative embodiment of the plunger 102 in its assembled form (i.e. when the top and bottom members 102a and 102b are held together or bonded together). In particular, Figures 50 to 58 respectively show the front view, left side view, right side view, back view, top view, bottom view, sectional view (along section A-A of Figure 42), upper isometric view and lower isometric view of a representative embodiment of the plunger 102. Figure 56 shows the representative configuration of the flanges for the embodiment shown in Figures 1 to 6.

At the locking position, the flanges 120 are shaped to receive the pins 110, 112, 114, 116 and 118 of the head portion 106 so as to prevent the head portion 106 from separating from the plunger 102 when the piston 108 moves in a rearward direction away from the plunger 102.

As shown in Figure 4, the recess 104 includes a shoulder 220, and the piston 108 includes a load bearing surface 222 for engaging the shoulder 220. The load bearing surface 222 is preferably shaped to sit flush against the shoulder 220, when the head portion 106 is fully received in the recess 104. The shoulder 220 and load bearing surface 222 provides a larger surface area on which the piston can exert force to push the plunger body 102 along the main chamber 200 of the syringe. The load bearing surface 222 is part of a support member 224. The support member 224 may be located behind the head portion 106. Preferably, the support member 224 positions the load bearing surface 222 at an angle that is substantially normal to the axis of rotation 124.

The support member 224 may include a sensor 226 for detecting the engagement and release of the head portion 106 from the plunger body 102. The sensor 226 may be a light sensor, or alternatively, the sensor 226 may be a combined infrared transceiver and receiver unit. The piston 108 includes hollow portions 228 that allow electrical and signal wiring to run to and from the sensor 226 to a control computer (not shown). The control computer receives signals from the sensor 226 representing whether the plunger body 102 is engaged or released (or separated) from the head portion 106 and plunger 102.

In a representative embodiment, the sensor 226 operates in conjunction with the outer wall 230 (or skirt) of the plunger body 102. Referring to the example shown in Figure 6, the sensor 226 is able to detect when the head portion 106 is separated from the plunger body 102, for example, by the absence of detecting any reflected infrared signal generated by the radiating element of the sensor 226. Accordingly, the sensor does not send a signal to the control computer, thus indicating that the plunger body 102 and head portion 106 are presently separated from each other. When the head portion 106 of the piston 108 is received in the plunger body 102, the outer wall 230 reflects at least some of the infrared signal generated by the sensor 226 back towards the detecting element of the sensor 226. As a result of this, the sensor 226 generates a signal to the control computer, thus indicating that the plunger body 102 and head portion 106 are coupled to each other.

The ability to detect whether the plunger body 102 is coupled to the head portion 106 can be important in some applications. For example, when the plunger body 102 is coupled to the head portion 106, it is undesirable to operate the piston in the withdrawal direction, which may result in the syringe withdrawing body fluid from a patient connected to the syringe. Also, the sensor 116 enables the control computer to control the lateral movement and strength exerted by the piston 108 and head portion 106, such as for engagement or disengagement with the plunger body 102 (with light forward or backward motion) or for exerting greater force to push the plunger body 102 along the syringe to inject material into a patient.

The outer wall 230 of the plunger body 102 is positioned proximate to an inner wall 204 of the syringe. The outer wall 230 has is sufficiently long in length so as to minimise skewing (e.g. lateral rotation) of the plunger body 102 relative to the inner wall 204 of the syringe 201 (e.g. when pushed along the syringe).

Figures 23 to 34 are diagrams of a representative example useful for understanding the invention of the connecting mechanism at different stages of operation. Figures 35 to 39 are diagrams of a rotatable head portion for that example.

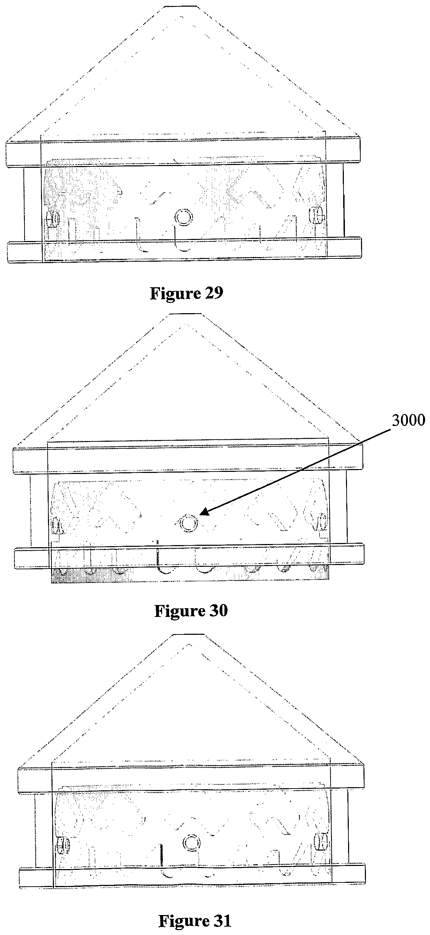

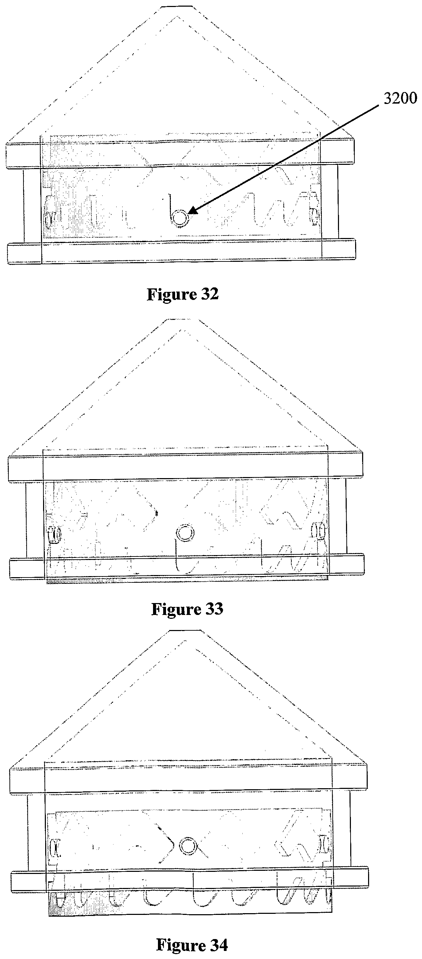

In the example useful for understanding the invention shown in Figures 23 to 39, the releasable connecting mechanism includes a piston 108 having a head portion 106 with one or more outwardly projecting flanges 3600. The head portion 106 is rotatable with the flanges 3600 about an axis along the piston 108. The plunger body 102 having a recess 104 formed within the plunger body 102 for receiving the head portion 106, the recess 104 including one or more inwardly projecting pins 2300 for guiding the rotation of the head portion 106 relative to the plunger body 106. Accordingly, when the head portion 106 is inserted into the recess 104, the pins 2300 rotate the head portion 106 to a locking position 3000 (see Figure 30) where the plunger body 102 resists disengagement from the piston 108. Also, when the head portion 106 is further inserted into the recess 104, the head portion 106 is released from the locking position 3000 and is allowed to rotate to a release position 3200 (see Figure 32) that allows the piston 108 to disengage from the plunger body 102.

The word 'comprising' and forms of the word 'comprising' as used in this description and in the claims does not limit the invention claimed to exclude any variants or additions.

Modifications and improvements to the invention will be readily apparent to those skilled in the art. Such modifications and improvements are intended to be within the scope of this invention.

## Claims

1. A releasable connecting mechanism for a syringe, said mechanism including:
a piston (108) having a head portion (106) with one or more radially projecting pins (110, 112, 114, 116, 118), said head portion (106) being rotatable with said pins about an axis (124) along said piston (108);
a plunger body (102) having a recess (104) formed within said body (102)
**characterized in that**
said recess (104) is adapted to receive said head portion and having one or more inwardly projecting flanges (120) for engaging and guiding said pins to rotate said head portion (106) relative to said body (102) within said recess (104);
wherein, when said head portion (106) is received into said recess (104), said flanges (3600) rotate said head portion (106) to a locking position (3000) where said body (102) resists disengagement from said piston (108), and when said head portion (106) is further inserted into said recess (104), said head portion (106) is released from said locking position (3000) and rotates to a release position (3200) allowing said piston (108) to disengage from said body (102).

2. A mechanism as claimed in claim 1, wherein said flanges (120) are shaped to guide the relative movement of said pins (110, 112, 114, 116, 118) along a saw tooth path in one direction.

3. A mechanism as claimed in claim 1, wherein, at said locking position, said flanges (120) are shaped to receive said pins (110, 112, 114, 116, 118) to prevent the separation of said piston (108) and said body (102) when said piston moves in a rearward direction away from said head portion (106).

4. A mechanism as claimed in claim 1, wherein:
said recess (104) includes a shoulder (220); and
said piston (108) includes a load bearing surface (222) for engaging said shoulder (220), said load bearing surface (222) being shaped to sit flush against said shoulder (220) when said head portion (106) is fully received in said recess (104).

5. A mechanism as claimed in claim 4 wherein said piston (108) including a support member (224) located behind said head portion (106), said load bearing surface (222) being a part of said support member (224) and being positioned at an angle substantially normal to said axis (124).

6. A mechanism as claimed in claim 1, wherein said piston (108) includes a sensor (226) for detecting the engagement and release of said piston (108) from said body (102).

7. A mechanism as claimed in claim 6, wherein said sensor (226) is a light sensor.

8. A mechanism as claimed in claim 5-4 and 5, wherein said sensor (226) is mounted to said support member (224) at an angle substantially normal to said axis (124).

9. A mechanism as claimed in claim 1, wherein said head portion (106) is coupled to a bearing (210) that is rotatably coupled to a pin (218) extending from an end portion of said piston (108), said bearing (210) forming a seal that minimises liquid from entering between said head portion (106) and said pin (218).

10. A mechanism as claimed in claim 1, wherein said plunger (102) body includes an outer wall that, when said plunger body (102) is fitted inside said syringe, is positioned proximate to an inner wall (204) of said syringe, said outer wall having sufficient length so as to minimise skewing of said plunger body (102) when pushed along said syringe.

11. A mechanism as claimed in claim 1, wherein the outer diameter of said head portion (106) is smaller than the outer diameter of said plunger body (102), such that when said head portion (106) is being received into said recess (104), said recess is shaped to push away from said head portion (106) any residue attached to the outer surface of said head portion (106).

12. A mechanism as claimed in claim 1, wherein said plunger body (102) includes:
a top concave member (102a) and
a bottom ring member (102b),
said top and bottom members being securely held together by a resilient cap (202) engaging an outer portion of said top and bottom members, said top member (102a) forming an interlocking engagement with said bottom member (102b) to minimise rotation of said top and bottom members relative to each other.

## Patentansprüche

1. Lösbarer Verbindungsmechanismus für eine Spritze, wobei der Mechanismus beinhaltet:
einen Kolben (108), der ein Kopfteil (106) mit einem oder mehreren radial vorspringenden Stiften (110, 112, 114, 116, 118) aufweist, wobei das Kopfteil (106) mit den Stiften drehbar um eine Achse (124) entlang des Kolbens (108) ist;
einen Stempelkörper (102), der eine Ausnehmung (104), die in dem Körper (102) geformt ist, aufweist,
**dadurch gekennzeichnet, dass**
die Ausnehmung (104) angepasst ist, das Kopfteil zu empfangen, und ein oder mehrere nach innen vorspringende Flansche (120) zum Eingreifen und Führen der Stifte zum Rotieren des Kopfteils (106) relativ zu dem Körper (102) innerhalb der Ausnehmung (104) aufweist;
wobei, wenn das Kopfteil (106) in der Ausnehmung (104) empfangen wird, die Flansche (3600) das Kopfteil (106) zu einer Verriegelungsposition (3000) rotieren, in der der Körper (102) einem Loslösen von dem Kolben (108) widersteht, und, wenn das Kopfteil (106) weiter in die Ausnehmung (104) eingebracht wird, das Kopfteil (106) aus der Verriegelungsposition (3000) freigegeben wird und zu einer Freigabeposition (3200) gedreht wird, die es erlaubt, den Kolben (108) von dem Körper (102) los zu lösen.

2. Mechanismus nach Anspruch 1, wobei die Flansche (120) so geformt sind, dass sie die relative Bewegung der Stifte (110, 112, 114, 116, 118) entlang eines Sägezahnpfades in eine Richtung führen.

3. Mechanismus nach Anspruch 1, wobei, in der Verriegelungsposition, die Flansche (120) so geformt sind, dass sie die Stifte (110, 112, 114, 116, 118) empfangen, um die Trennung des Kolbens (108) und des Körpers (102) zu verhindern, wenn sich der Kolben in einer rückwärtigen Richtung weg von dem Kopfteil (106) bewegt.

4. Mechanismus nach Anspruch 1, wobei:
die Ausnehmung (104) eine Schulter (220) beinhaltet; und
der Kolben (108) eine lasttragende Oberfläche (222) zum Eingreifen der Schulter (220) beinhaltet, wobei die lasttragende Oberfläche (222) so geformt ist, dass sie glatt gegen die Schulter (220) sitzt, wenn das Kopfteil (106) vollständig in der Ausnehmung (104) empfangen ist.

5. Mechanismus nach Anspruch 4, wobei der Kolben (108) ein Stützelement (224) beinhaltet, das hinter dem Kopfteil (106) angeordnet ist, wobei die lasttragende Oberfläche (222) ein Teil des Stützelements (224) ist und in einem Winkel positioniert ist, der im Wesentlichen senkrecht auf der Achse (124) steht.

6. Mechanismus nach Anspruch 1, wobei der Kolben (108) einen Sensor (226) zum Detektieren des Eingreifens und des Loslösens des Kolbens (108) von dem Körper (102) beinhaltet.

7. Mechanismus nach Anspruch 6, wobei der Sensor (226) ein Lichtsensor ist.

8. Mechanismus nach einem der Ansprüche 4 und 5, wobei der Sensor (226) an dem Stützelement (224) unter einem Winkel befestigt ist, der im Wesentlichen senkrecht zu der Achse (124) ist.

9. Mechanismus nach Anspruch 1, wobei das Kopfteil (106) mit einem Lager (210) gekoppelt ist, das drehbar mit einem Stift (218) gekoppelt ist, der sich von einem Endteil des Kolbens (108) aus erstreckt, wobei das Lager (210) eine Dichtung bildet, die das Eindringen von Flüssigkeit zwischen dem Kopfteil (106) und dem Stift (218) minimiert.

10. Mechanismus nach Anspruch 1, wobei der Stempelkörper (102) eine äußere Wand beinhaltet, die, wenn der Stempelkörper (102) in die Spritze eingesetzt ist, benachbart zu einer inneren Wand (204) der Spritze positioniert ist, wobei die äußere Wand eine ausreichende Länge hat, um ein Verkippen des Stempelkörpers (102) zu minimieren, wenn dieser entlang der Spritze gedrückt wird.

11. Mechanismus nach Anspruch 1, wobei der äußere Durchmesser des Kopfteils (106) kleiner ist als der äußere Durchmesser des Stempelkörpers (102), sodass, wenn das Kopfteil (106) in der Ausnehmung (104) empfangen wird, die Ausnehmung so geformt ist, dass sie jegliche Rückstände, die an der äußeren Oberfläche des Kopfteils (106) anhaften, von dem Kopfteil (106) wegschiebt.

12. Mechanismus nach Anspruch 1, wobei der Stempelkörper (102) beinhaltet:
ein oberes konkaves Element (102a) und
ein unteres Ringelement (102b),
wobei die oberen und unteren Elemente sicher durch eine elastische Kappe (202) zusammengehalten werden, welche in einen äußeren Teil der oberen und unteren Elemente eingreift, wobei das obere Element (102a) eine ineinander eingreifende Kupplung mit dem unteren Element (102b) bildet, um eine Drehung der oberen und unteren Elemente relativ zueinander zu minimieren.

## Revendications

1. Mécanisme de liaison amovible pour une seringue, ledit mécanisme comprenant:
un piston (108) ayant une partie de tête (106) avec une ou plusieurs broches (110, 112, 114, 116, 118) faisant saillie radialement, ladite partie de tête (106) étant apte à tourner avec lesdites broches autour d'un axe (124) le long dudit piston (108);
un corps plongeur (102) présentant un évidement (104) qui est formé à l'intérieur dudit corps (102),
**caractérisé par le fait que**
ledit évidement (104) est adapté pour recevoir la partie de tête et comprend une ou plusieurs brides (120) qui font saillie vers l'intérieur afin d'engager et de guider lesdites broches pour faire tourner ladite partie de tête (106) par rapport au corps (102) à l'intérieur de l'évidement (104);
dans lequel, lorsque la partie de tête (106) est reçue à l'intérieur de l'évidement (104), lesdites brides (3600) font tourner la partie de tête (106) jusqu'à une position de verrouillage (3000) dans laquelle ledit corps (102) résiste à son détachement dudit piston (108), et, lorsqu'on insère encore plus la partie de tête (106) dans l'évidement (104), la partie de tête (106) est libérée de la position de verrouillage (3000) et effectue une rotation jusqu'à une position de libération (3200) permettant au piston (108) de se détacher du corps (102).

2. Mécanisme selon la revendication 1, dans lequel lesdites brides (120) sont formées de manière à guider le mouvement relatif desdites broches (110, 112, 114, 116, 118) le long d'un trajet de dents de scie dans une direction.

3. Mécanisme selon la revendication 1, dans lequel, dans la position de verrouillage, lesdites brides (120) sont formées de manière à recevoir les broches (110, 112, 114, 116, 118) afin d'empêcher la séparation dudit piston (108) et dudit corps (102) lorsque le piston se déplace dans une direction arrière en s'éloignant de la partie de tête (106).

4. Mécanisme selon la revendication 1, dans lequel:
ledit évidement (104) comprend un épaulement (220); et
ledit piston (108) comprend une surface de support de charge (222) destinée à engager ledit épaulement (220), ladite surface de support de charge (222) étant formée de manière à reposer à plat contre ledit épaulement (220) lorsque la partie de tête (106) est complètement reçue dans l'évidement (104).

5. Mécanisme selon la revendication 4, dans lequel ledit piston (108) comprend un élément de support (224) agencé derrière la partie de tête (106), ladite surface de support de charge (222) étant une partie dudit élément de support (224) et étant positionnée selon un angle pour l'essentiel perpendiculaire à l'axe (124).

6. Mécanisme selon la revendication 1, dans lequel ledit piston (108) comprend un capteur (226) pour détecter l'engagement et le détachement dudit piston (108) par rapport audit corps (102).

7. Mécanisme selon la revendication 6, dans lequel ledit capteur (226) est un capteur de lumière.

8. Mécanisme selon l'une quelconque des revendications 4 et 5, dans lequel ledit capteur (226) est fixé sur l'élément de support (224) selon un angle pour l'essentiel perpendiculaire audit axe (124).

9. Mécanisme selon la revendication 1, dans lequel ladite partie de tête (106) est couplée à un palier (210) qui est couplé de manière rotative à une broche (218) s'étendant depuis une partie d'extrémité dudit piston (108), ledit palier (210) formant un joint d'étanchéité qui minimise l'entrée de liquide entre ladite partie de tête (106) et ladite broche (218).

10. Mécanisme selon la revendication 1, dans lequel ledit corps plongeur (102) comprend une paroi extérieure qui, lorsque ledit corps plongeur (102) est ajusté à l'intérieur de ladite seringue, est positionnée à proximité d'une paroi intérieure (204) de ladite seringue, ladite paroi extérieure ayant une longueur suffisante afin de minimiser une inclinaison dudit corps plongeur (102) lorsqu'il est poussé le long de la seringue.

11. Mécanisme selon la revendication 1, dans lequel le diamètre extérieur de ladite partie de tête (106) est inférieur au diamètre extérieur dudit corps plongeur (102) de sorte que, lorsque ladite partie de tête (106) est reçue dans ledit évidement (104), l'évidement est formé de manière à repousser de la partie de tête (106) tout résidu adhérant sur la surface extérieure de la partie de tête (106).

12. Mécanisme selon la revendication 1, dans lequel ledit corps plongeur (102) comprend:
un élément concave supérieur (102a) et
un élément annulaire inférieur (102b),
lesdits éléments supérieur et inférieur étant maintenus ensemble de manière sûre par un capuchon (202) élastique qui s'engage dans une partie extérieure des éléments supérieur et inférieur, ledit élément supérieur (102a) formant un accouplement par enclenchement avec ledit élément inférieur (102b) pour minimiser une rotation des éléments supérieur et inférieur l'un par rapport à l'autre.
